# DEMANDE DE BREVET EUROPEEN

(11) **EP 0 558 392 A1**
(43) Date de publication de la demande: **01.09.1993**
(21) Numéro de dépôt: 93400446.6
(22) Date de dépôt: 22.02.1993
(51) Int. Cl.: C07D 217/10, H01G 9/02

(54) **Procédé de stabilisation d'un complexe à transfert de charge basé sur le 7,7,8,8-tétracyanoquinodiméthane**

(30) Priorité: 28.02.1992 FR 9202366
(71) Demandeur: COMPAGNIE EUROPEENNE DE COMPOSANTS ELECTRONIQUES LCC, F-92400 Courbevoie (FR)
(72) Inventeur: Michel, Philippe, F-92402 Courbevoie Cedex (FR); Sagnes, Olivier, F-92402 Courbevoie Cedex (FR); Poupard, Dominique, F-92402 Courbevoie Cedex (FR); Westland, Duncan James, F-92402 Courbevoie Cedex (FR); Skarda, Vladimir, F-92402 Courbevoie Cedex (FR); Staight, John, F-92402 Courbevoie Cedex (FR); Arena Francesco, F-92402 Courbevoie Cedex (FR); Zanellato Fabrizio, F-92402 Courbevoie Cedex (FR)
(74) Mandataire: Guérin, Michel

(57) **Abrégé**

La présente invention concerne un procédé de stablilisation d'un complexe à transfert de charge basé sur le 7, 7 ,8, 8-tétracyanoquinodiméthane (généralement référencé TCNQ).

Dans ce cas, le complexe à transfert de charge est mélangé avec du TCNQ neutre et le mélange est mis en phase fondue.

Application notamment dans le domaine des condensateurs électrolytiques.

## Description

La présente invention concerne un procédé de stabilisation d'un complexe à transfert de charge basé sur le 7, 7, 8, 8-tétracyanoquinodiméthane (généralement référencé TCNQ). La présente invention concerne aussi certains sels à transfert de charge à base de TCNQ utilisés plus particulièrement dans ce procédé de stabilisation.

Depuis quelques années, dans la fabrication des condensateurs électrolytiques, on cherche à utiliser comme électrolyte solide, des sels à transfert de charge à base de 7, 7, 8, 8-tétracyanoquinodiméthane. Ces sels à transfert de charge, lorsqu'ils sont utilisés dans la fabrication de condensateurs électrolytiques, donnent des condensateurs présentant une réponse à fréquence élevée stable et une densité de courant de fuite très faible. Toutefois, ces sels présentent une faible stabilité thermique, notamment lorsqu'ils sont en phase fondue. Or, la mise en oeuvre des sels nécessite généralement une étape où ils sont utilisés dans cette phase. Dans ce cas, on observe une dégradation rapide du produit, ce qui limite son utilisation. Or, les sels à transfert de charge doivent être suffisamment stables thermiquement pour pouvoir être utilisés dans des condensateurs électrolytiques soumis à des traitements thermiques importants, tels que les condensateurs-chips destinés à un montage en surface. Dans ce cas, les condensateurs doivent pouvoir soutenir une température de 260°C pendant 10 secondes, ce qui correspond au temps de la soudure du montage en surface. Il est donc nécessaire de pouvoir disposer de sels dont la température de fusion soit voisine de 260°C. Or, le traitement des sels à transfert de charge à base de TCNQ à des températures supérieures à leurs points de fusion présente des problèmes puisque la plupart des sels connus se décomposent au point de fusion ou à une température légèrement supérieure au point de fusion.

En conséquence, la présente invention a pour but de proposer un procédé permettant de stabiliser les sels à transfert de charge à base de TCNQ en augmentant la différence entre la température de dégradation et la température de fusion, ce qui augmente la stabilité des sels dans leur phase fondue. Il est ainsi possible de les utiliser, notamment dans la fabrication des condensateurs à électrolyte solide. La présente invention concerne aussi des nouveaux sels à transfert de charge à base de TCNQ qui peuvent être utilisés dans ce procédé de stabilisation et qui présentent l'avantage d'être à la fois fusibles et stables thermiquement.

En conséquence, la présente invention a pour objet un procédé de stabilisation d'un complexe à transfert de charge basé sur le 7, 7, 8 ,8-tétracyanoquinodiméthane (généralement référencé TCNQ), caractérisé en ce que le dit complexe à transfert de charge est mélangé avec du TCNQ neutre et en ce que le mélange est mis en phase fondue.

Selon un mode de réalisation préférentiel, la fusion est réalisée sous atmosphère d'oxygène, sous atmosphère d'azote ou éventuellement sous atmosphère neutre, à savoir sous atmosphère d'un gaz rare tel que l'argon ou similaire.

D'autre part, selon une caractéristique supplémentaire de la présente ivention, lorsque la fusion est réalisée sous oxygène, on ajoute plus de 100 % en poids de TCNQ neutre pour obtenir une différence entre la température de dégradation et la température de fusion de 20°C. Toutefois, lorsque la fusion est réalisée sous azote, on ajoute jusqu'à 25 % en poids de TCNQ neutre pour obtenir une différence entre la température de dégradation et la température de fusion de 20°C. Ainsi, pour obtenir une stabilité importante, il est préférable de réaliser la mise en phase fondue du mélange sous atmosphère d'azote.

La présente invention a aussi pour objet des sels à transfert de charge à base de TCNQ utilisés principalement dans le procédé de stabilisation décrit ci-dessus et caractérisé en ce qu'ils sont donnés par la formule : 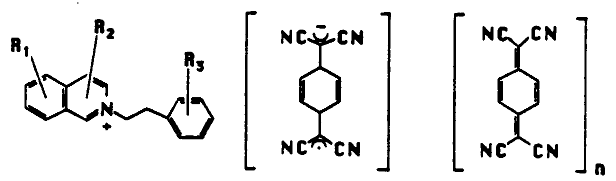
dans laquelle 0 < n ≦ 2 et R1, R2, R3 qui peuvent être identiques ou différents représentent chacun de l'hydrogène ou un groupe alkyl linéaire ou ramifié qui peut être substitué ou un noyau aromatique condensé ou non.

Selon un mode de réalisation préférentiel, les sels sont choisis notamment parmi le phényléthylisoquinolinium (TCNQ)₂ et le naphtyléthylisoquinolinium (TCNQ)₂.

D'autres caractéristiques et avantages de la présente invention apparaîtront à la lecture de la description de différents modes de réalisation du procédé de stabilisation, cette description étant faite avec référence aux dessins ci-annexés dans lesquels :
- la figure 1 représente la différence entre la température de fusion et la température de dégradation du phényléthylisoquinolinium (TCNQ)₂ en fonction du pourcentage de TCNQ neutre ajouté lorsque la fusion est réalisée sous atmosphère d'oxygène,
- la figure 2 est une courbe donnant la température de fusion et la température de dégradation du phényléthylisoquinolinium (TCNQ)₂ en fonction du pourcentage de TCNQ neutre ajouté lorsque la fusion est réalisé sous atmosphère d'azote,
- la figure 3 est une courbe donnant le temps disponible avant décomposition du phényléthylisoquinolinium (TCNQ)₂ dans la phase fondue en fonction du pourcentage de TCNQ neutre ajouté sous atmosphère d'oxygène,

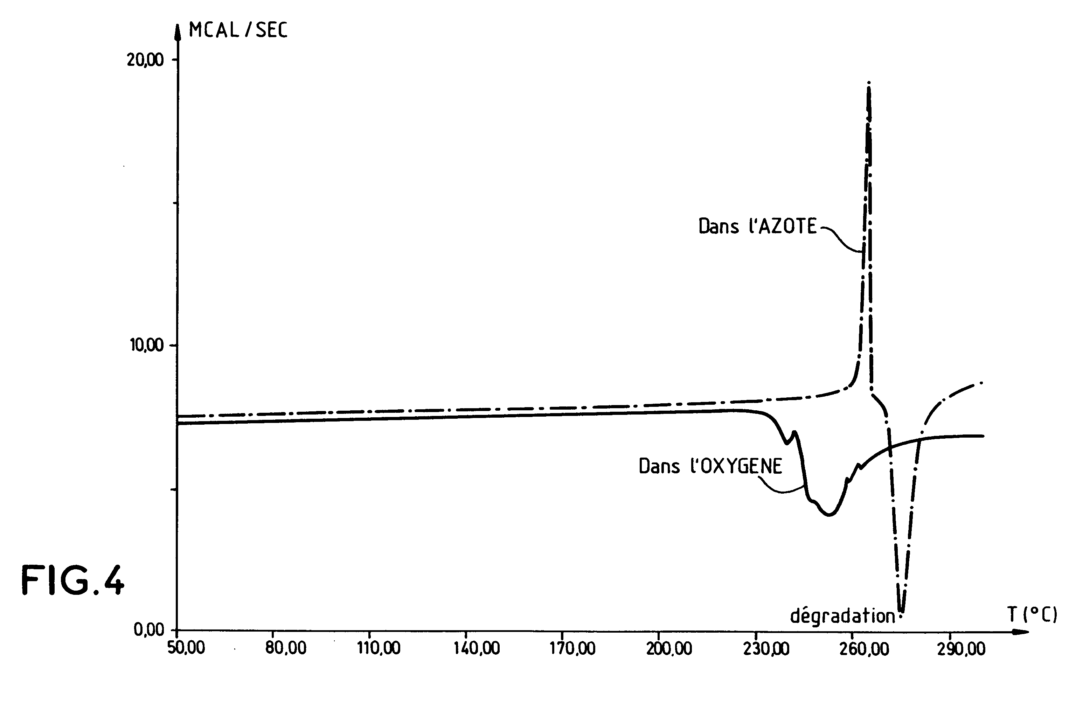

- la figure 4 donne les résultats de l'analyse thermique différentielle du phényléthylisoquinolinium (TCNQ)₂ et,
- la figure 5 est une courbe donnant la température de fusion et la température de dégradation du naphtylethylisoquinolinium (TCNQ)₂ en fonction du pourcentage de TCNQ neutre ajouté sous atmosphère d'azote.

Le procédé de stabilisation de la présente invention sera décrit à l'aide de différents exemples utilisant différents complexes à transfert de charge basés sur le 7,7,8,8-tétracyanoquinodiméthane et en réalisant la fusion soit sous atmosphère d'oxygène, soit sous atmosphère d'azote.

### Exemple 1 :

Dans cet exemple, ce procédé de stabilisation sera décrit en utilisant comme complexe à transfert de charge à base de TCNQ, le phényléthylisoquinolinium (TCNQ)₂ , référencé ci-après PEI (TCNQ)₂.

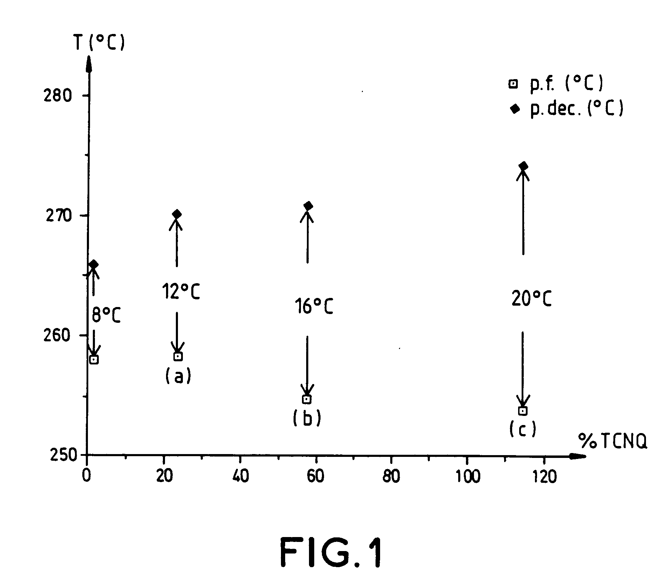

Dix milligrammes de PEI (TCNQ)₂ ont été mélangés avec, respectivement, 2,18 milligrammes (exemple a), 5,59 milligrammes (exemple b) et 11,26 milligrammes (exemple c) de TCNQ puis finement broyés. Le mélange a alors été mis en phase fondue. La fusion a été réalisée sous atmosphère d'oxygène. Une analyse thermique différentielle des différents mélanges mis en phase fondue montre que l'addition d'une quantité croissante de TCNQ neutre abaisse la température de fusion et augmente la température de dégradation, comme représenté sur la figure 1. Ainsi, par exemple, dans le cas où plus de 100 % en poids de TCNQ neutre est ajouté au PEI (TCNQ)₂ , une différence de température de 20°C est obtenue. Or, pour un produit standard sans addition de TCNQ neutre, une différence d'environ 8°C peut être obtenue sous oxygène comme réprésenté sur la figure 1.

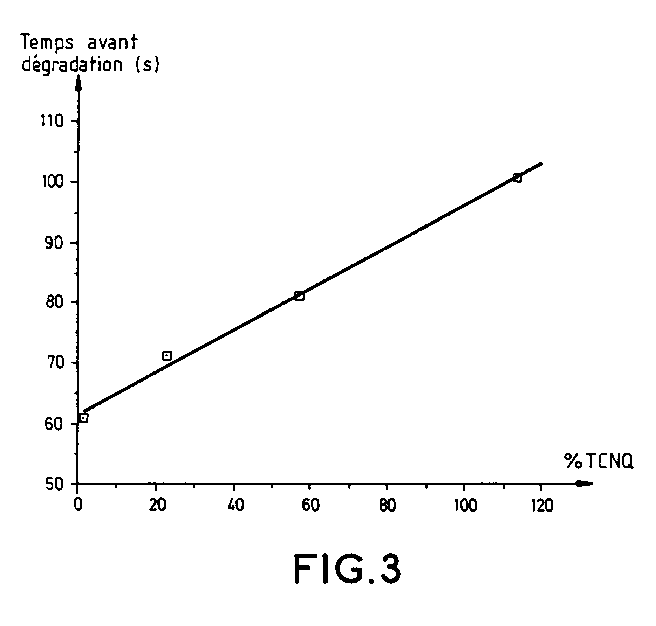

D'autre part, le graphe de la figure 3 obtenu lors d'une analyse thermogravimétrique (TGA) qui concerne le temps disponible avant décomposition de phényléthylisoquinolinium (TCNQ)₂ en phase fondue en fonction de l'ajout de TCNQ neutre sous atmosphère d'oxygène montre deux caractéristiques importantes. Tout d'abord, l'intervalle de temps entre la fusion et la dégradation du produit augmente avec la quantité de TCNQ ajouté et peut atteindre une valeur de 105 secondes. Ceci permet d'augmenter le temps pendant lequel on peut utiliser le mélange, notamment dans l'imprégnation d'anodes pour condensateurs électrolytiques. D'autre part, une analyse élémentaire des échantillons dégradés montre qu'une grande quantité de TCNQ ajouté est toujours présente après dégradation. On suppose donc que l'effet de stabilisation est dû à la dissolution de TCNQ neutre en excès dans le matériau fondu qui compense la perte de TCNQ sublimé du matériau lui-même , sublimation qui a lieu au cours de la fusion du produit.

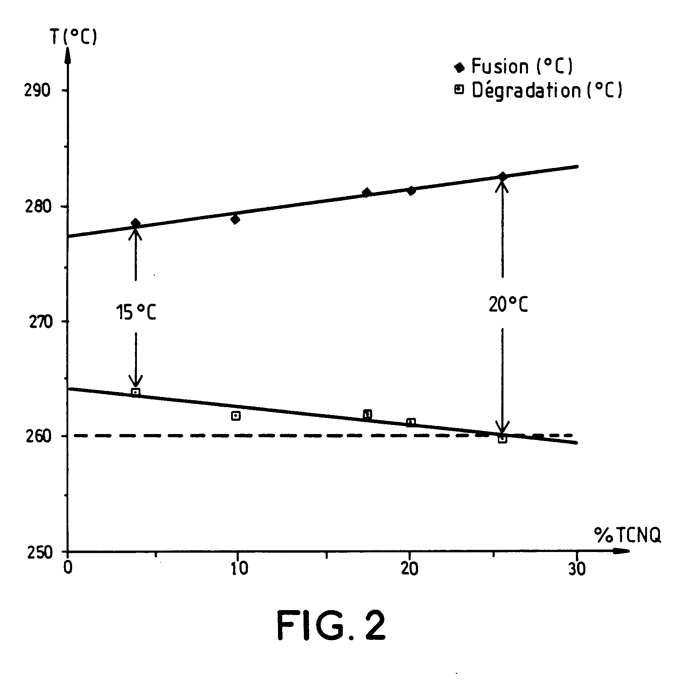

Lorsque la fusion du mélange PEI (TCNQ)₂ et TCNQ neutre est réalisée sous atmosphère d'azote, on obtient des résultats très intéressants au niveau de la stabilisation. En effet, comme représenté sur la figure 2, le pourcentage de TCNQ neutre qui doit être ajouté pour obtenir, par exemple, une différence de température de 20°C, est bien inférieur à celui qui doit être ajouté lorsqu'on travaille sous atmosphère d'oxygène. Sur la figure 2, on s'aperçoit que pour 25 % en poids de TCNQ neutre ajouté, on obtient une différence de température de 23°C.

### Exemple 2 :

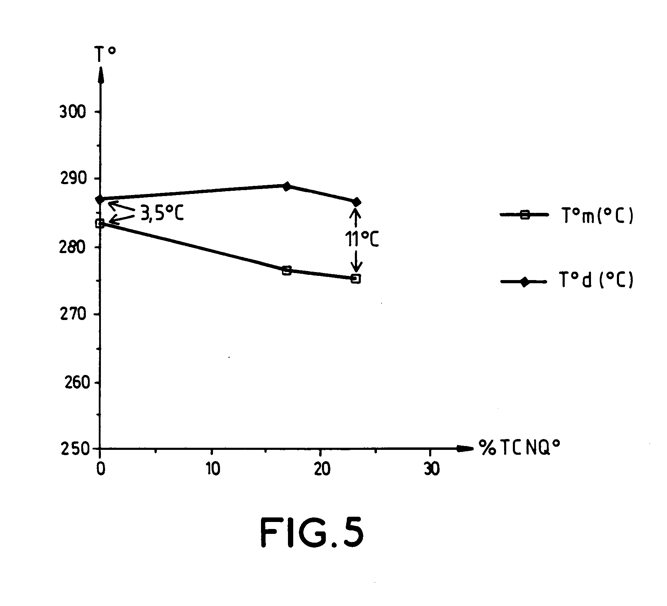

Dans ce cas, la stabilisation a été effectuée en utilisant comme sel à transfert de charge basé sur le TCNQ, le naphtyléthylisoquinolinium (TCNQ)₂ référencé ci-après NEI (TCNQ)₂ . 100 mg de NEI (TCNQ)₂ ont été mélangés avec respectivement 16 mg puis 23 mg de TCNQ neutre. Le mélange obtenu est mis en phase fondue sous azote 24 heures avant la mesure. Dans ce cas, l'on obtient les résultats représentés sur la figure 5 qui correspond à la figure 3.

Le procédé de stabilisation de la présente invention peut être mis en oeuvre en utilisant tous types de sels à transfert de charge fusibles basés sur du TCNQ. Toutefois, il est particulièrement utile avec des sels complexes à transfert de charge à base de TCNQ fusibles et stables thermiquement correspondant à la formule générale suivante : 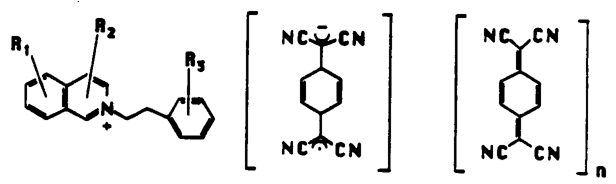
dans laquelle 0 < n ≦ 2 et R1, R2, R3, qui peuvent être identiques ou différents, représentent de l'hydrogène ou des groupes alkyls linéaires ou ramifiés qui peuvent être substitués ou des noyaux aromatiques condensés ou non. De tels sels présentent l'avantage d'avoir une température de fusion supérieure à 260°C, ce qui permet leur utilisation, notamment, dans la fabrication de condensateurs à électrolyte solide de type chips destinés au montage en surface.

On décrira ci-après un mode de réalisation de la synthèse d'un sel complexe à transfert de charge du type de celui décrit ci-dessus, en se référant plus particulièrement au phényléthylisoquinolinium (TCNQ)₂ comme exemple.

De manière typique, 2,2 grammes d'iodure de phényléthylisoquinolinium ont été dissouts dans 150ml d'acétonitrile chaud sous atmosphère d'azote. La solution résultante a été ajoutée à une solution sous ébullition de 1, 85 grammes de TCNQ dans 210ml du même solvant sous azote. Le mélange réactionnel a été chauffé au reflux pendant trois heures. Le PEI (TCNQ)₂ cristallin de couleur vert foncé résultant a été filtré, lavé avec de l'éthyl-éther et séché sous vide. Ce produit a été caractérisé par spectrométries infra-rouge et visible. Le spectre visible est typique de celui d'un sel complexe où des anions radicaux de TCNQ et du TCNQ neutre sont simultanément présents. Le spectre d'absorption électronique des sels en solution présente une absorption qui est essentiellement une sommation des bandes caractéristiques du TCNQ neutre et de l'anion TCNQ. Pour les sels complexes, le rapport d'intensité des bandes 392nm et 842nm fournit un moyen commode pour une première détermination de la composition du sel. On a aussi mesuré la conductivité du sel et on a obtenu une valeur de 0,43 S cm⁻¹. L'analyse thermique différentielle du PEI (TCNQ)₂ présente une température de fusion de 260°C et une température de dégradation de 275°C . Ce matériau présente donc une différence entre la température de dégradation et la température de fusion d'environ 15°C sous azote et peut être maintenu dans l'état fondu pendant un temps relativement long sans décomposition, ce qui permet son utilisation notamment dans la fabrication de condensateurs à électrolyte solide. D'autre part, on a préparé plusieurs exemples dans le but de contrôler le rapport du TCNQ neutre au TCNQ resté à l'intérieur du sel lui-même. Dans ce but, différentes stoëchiométries ont été utilisées pour la synthèse des matériaux. Le tableau 1 représente les données collectées pour les différents exemples. Les exemples 1, 2, 4 et 5 du tableau montrent qu'en utilisant une procédure de synthèse identique, il est possible de reproduire le même ratio d'absorbance. D'autre part, il apparaît à partir du tableau que des stoëchiométries différentes peuvent donner le même rapport spectrométrique.

| **Exemple** | **PEII/TCNQ** | **A₃₉₄/A₈₄₂** | **T**_{**f**} **(°C)** | **T**_{**d**} **(°C)** | **o (S.cm⁻¹)** |
|---|---|---|---|---|---|
| **Exp.1** | 1:2 | 1,94 | 264 | 275 | 0,72 |
| **Exp.** **2** | 1:2 | 1,91 | 261 | 269 | --- |
| **Exp.** **3** | 1:1.5 | 1,86 | 262 | 275 | 0,43 |
| **Exp.** **4** | 1:3 | 1,72 | --- | --- | 0,34 |
| **Exp.** **5** | 1:1 | 1,75 | 263 | 275 | 0,34 |
| **Exp.** **6** | 1:1 | 1,74 | --- | --- | 0,30 |

## Revendications

1. Procédé de stablilisation d'un complexe à transfert de charge basé sur le 7, 7 , 8, 8-tétracyanoquinodiméthane (généralement référencé TCNQ), caractérisé en ce que ledit complexe à transfert de charge est mélangé avec du TCNQ neutre et en ce que le mélange est mis en phase fondue.

2. Procédé selon la revendication 1, caractérisé en ce que la fusion est réalisée sous oxygène, sous azote ou sous atmosphère neutre.

3. Procédé selon l'une quelconque des revendications 1 et 2, caractérisé en ce que, lorsque la fusion est réalisée sous oxygène, on ajoute plus de 100% en poids de TCNQ neutre pour obtenir une différence entre la température de dégradation et la température de fusion de 20°C.

4. Procédé selon l'une quelconque des revendications 1 et 2, caractérisé en ce que, lorsque la fusion est réalisée sous azote, on ajoute jusqu'à 25% en poids de TCNQ neutre pour obtenir une différence entre la température de dégradation et la température de fusion de 20°C.

5. Procédé selon l'une quelconque des revendications 1 à 4, caractérisé en ce que le mélange fondu est utilisé pour l'imprégnation d'un condensateur à électrolyte solide.

6. Sels à transfert de charge à base de TCNQ utilisés dans le procédé de stabilisation selon l'une quelconque des revendications 1 à 5, caractérisé en ce qu'ils sont donnés par la formule 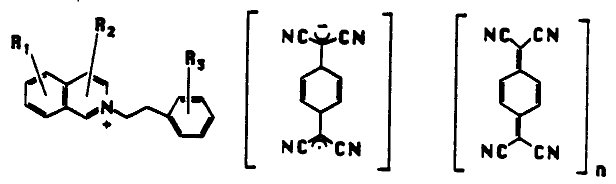 dans laquelle 0 < n ≦ 2 et R1, R2, R3 qui peuvent être identiques ou différents représentent chacun de l'hydrogène ou un groupe alkyle linéaire ou ramifié qui peut être substitué ou un noyau aromatique condensé ou non.

7. Sels selon la revendication 6, caractérisés en ce qu'ils comportent le phényléthylisoquinolinium (TCNQ)₂, le naphtylethylisoquinolinium (TCNQ)₂.
